# EUROPEAN PATENT SPECIFICATION

(11) **EP 2 563 215 B1**
(45) Date of publication and mention of the grant of the patent: **02.03.2016**
(21) Application number: 10719742.8
(22) Date of filing: 26.04.2010
(51) Int. Cl.: A61B 5/053, A61B 5/00

(54) **METHOD AND DEVICE FOR QUALITY ASSESSMENT OF AN ELECTRICAL IMPEDANCE MEASUREMENT ON TISSUE**
VERFAHREN UND VORRICHTUNG ZUR QUALITÄTSBEURTEILUNG EINER ELEKTRISCHEN IMPEDANZMESSUNG AUF GEWEBE
PROCÉDÉ ET DISPOSITIF POUR ÉVALUER LA QUALITÉ D'UNE MESURE D'IMPÉDANCE ÉLECTRIQUE SUR UN TISSU

(43) Date of publication of application: 06.03.2013
(73) Proprietor: SciBase AB, 103 67 Stockholm (SE)
(72) Inventor: ÅBERG, Peter, S-122 46 Enskede (SE); GOLDKUHL, Fredrik, S-172 37 Sundbyberg (SE); DALMAU, Jörgen, S-164 72 Kista (SE)
(74) Representative: Groth & Co. KB
(86) International application number: PCT/EP2010/055535
(87) International publication number: WO 2011/134489

(56) References cited:
- US-A- 5 921 939
- US-A1- 2003 006 782
- US-A1- 2003 088 185
- US-A1- 2007 038 257
- US-A1- 2009 076 350
- US-A1- 2009 299 422
- ABERG P ET AL: "Skin Cancer Identification Using Multifrequency Electrical Impedance-A Potential Screening Tool", IEEE TRANSACTIONS ON BIOMEDICAL ENGINEERING, IEEE SERVICE CENTER, PISCATAWAY, NJ, US, vol. 51, no. 12, 1 December 2004 (2004-12-01), pages 2097-2102, XP011122294, ISSN: 0018-9294, DOI: DOI:10.1109/TBME.2004.836523

## Description

### Field of the Invention

The present disclosure generally relates to the diagnosis, determination, characterization or assessment of biological conditions, e.g. diseased conditions, in tissue of a human, animal or other subject. Particularly, the present invention relates to assessment of tissue by means of electrical impedance data.

### Background of the Invention

Skin cancer is a rapidly increasing form of cancer in many countries throughout the world. The most common form of skin cancers are basal cell carcinoma, squamous cell carcinoma, and melanoma. Melanoma is one of the rarer types of skin cancer but causes the majority of skin cancer related deaths. It has been suggested that the majority of skin cancer cases are caused by too much exposure to sunlight. As with other types of cancer, it is important that skin cancer, especially melanoma, is diagnosed at such an early stage as possible.

However, clinical diagnosis of skin tumours may prove difficult even for experienced dermatologists, especially in the case of malignant melanoma. Thus, there is an increasing need for a diagnostic aid besides the established method of employing ocular inspections in combination with skin biopsies for histological examination.

Electrical impedance imaging has been proposed to form an image of electrical impedance differences within a body region. It is noted that the image does not necessarily need to correspond to an actual image of an abnormal condition, e.g., a lesion, but may rather be construed broadly as a pattern that may be used for identifying such abnormal conditions. However, the separation of diseased tissue, such as malignant tumours, from healthy tissue or merely mildly diseased tissue (e.g., benign lesions) based on impedance measurements needs further investigation. In this regard, there are fundamental problems that need to be addressed when trying to construct an image or pattern from impedance data. For one thing, electrical currents within the body follow the path of least resistance, in general being an irregular path not restricted to a particular line or even a plane in the body, which may be an issue in reconstructing the spatial distribution of electrical properties in the body from impedance data. Furthermore, electrical impedance data obtained from impedance measurements in tissue is multivariate and further comprises complex numbers, comprising magnitude and phase. Notwithstanding the problem of analyzing complex numbers, such multivariate data further generally represents a very large data set which may be cumbersome to analyze, even with powerful data processing means.

In order to obtain reliable and reproducible tissue electrical impedance data, it may be important that the impedance measurement is performed correctly to minimize the source of error. Further, an impedance analysis may include an impedance measurement of healthy, reference tissue to be compared with an impedance measurement of tissue suspected to be diseased. This implies that not only has a correct measurement of the suspected diseased tissue be performed, but also a correct measurement of the reference tissue, to obtain reliable data.

Examples of prior art documents showing various devices for electrical impedance measurements using filtering algorithms are following.

Document US 2007/0038257 describes an electrode connection quality detection apparatus for determining the electrode connection quality or interface quality at biomedical measurements. This apparatus does not have an acral filter.

US 2009/0076350: describes a system for tracking a patient's physiological status including a monitoring system that detect events of the sensors such as high noise states, low noise states, and sensor compliance and performance.

US 2009/0299422: describes an implantable device that stores EGM data based on whether sensing integrity criterion is met, e.g. whether an impedance criterion has been met. The impedance criterion may indicate sensing integrity criterions, for example, a lead related criterion such as lead fracture or short.

US 5,921,939 describes a device for monitoring measurement electrodes attached to a patient by continuously monitoring a pure AC signal, i.e. a test signal, sent to the tissue of the patient via neutral electrode. By monitoring the test signal it is possible to verify whether the measurement electrodes are in contact with the patient.

US 2003/0088185 describes a method and apparatus for sensing and/or recording electrical activity in the nerve tissue including detection of probe contact with patient tissue. The detection of probe contact is achieved, for example, by continuous measurements of stimulation circuit impedance and measurements of current flow in the circuit. US 2003/0006782 describes a system for measuring bioelectric impedance in real time in presence of interference and noise. The impedance measurements are tested for contamination by applying a SNR threshold to the measured impedance.

### Summary of the Invention

It is an objective of the present invention to improve the possibility of diagnosing a tissue condition by means of electrical impedance measurement.

This objective, as well as other objectives that will be apparent from the following, is achieved through a method and a device in accordance with the appended independent claims.

According to one aspect of the present disclosure there is provided a method of assessing the quality of an electrical impedance measurement on tissue of a subject, the method comprising: performing the impedance measurement on a tissue region of said tissue of the subject, whereby impedance data is obtained, said data comprising at least one impedance value measured in said tissue region; applying an evaluation algorithm to the obtained impedance data, whereby the quality of the impedance measurement is assessed; and presenting the assessed quality of the impedance measurement such that a decision can be made on whether to use the impedance measurement for diagnosing a condition of said tissue of the subject.

The impedance measurement may be performed in any way that is adequate to obtain an impedance value of the tissue, such as measuring the impedance between two or more electrodes placed against a surface of the tissue or inserted into the tissue. The impedance measurement may be performed with a device for electrical impedance measurement on tissue.

The impedance data comprises at least one impedance value of the tissue region. It may be convenient to allow the impedance data to comprise a plurality of impedance values. A plurality of impedance values may improve the accuracy and usability of the data.

The impedance value(s) comprised in the impedance data may comprise the magnitude and/or the phase of measured impedance. The magnitude and/or phase may be measured at any AC frequency or at a plurality of frequencies, or the magnitude and/or phase may be essentially continuously measured in a continuous or discontinuous frequency spectrum between end frequencies. Thus, the impedance data may comprise impedance values of different frequencies, increasing the impedance information comprised in the impedance data which may be used for assessment and diagnosing.

The impedance data may comprise impedance values, such as magnitude and/or phase, relating to different depths of the tissue. This may be achieved e.g. by inserting measurement electrodes to different depths of the tissue or, non-invasively, by measuring between electrodes placed against the surface of the tissue at different distances from each other. If the electrodes are placed further from each other, the impedance of a larger tissue volume may be measured. When two electrodes are placed further from each other, the measured volume expands not only along an imagined straight line between the electrodes, but also perpendicular to this line. Thus, the impedance of a tissue may be measured to different depths of the tissue by employing electrodes placed against a surface of the tissue at different distances from each other. These measurements at/to different depths may be made concurrently or sequentially. Thus, the impedance data may comprise impedance values at/to different tissue depths, increasing the amount of impedance information comprised in the impedance data which may be used for assessment and diagnosing.

The assessed quality of the impedance measurement may be presented in any way that allows a decision to be made on whether to use the impedance measurement for diagnosing a condition of the tissue. It may e.g. be presented to a computer or other automated or pre-programmed means for making the decision or be presented to a human operator. The human operator may e.g. be a physician, a nurse, any other hospital or care facility staff, or an engineer. The operator may e.g. be a person responsible for performing the impedance measurement with a device for electrical impedance measurement on tissue or be a person only responsible for perceiving the assessed quality. The presenting may e.g. be made with sound, white or coloured light, vibration or with a display able to display symbols such as numbers, letters and signs or be made in any other way which may be perceived by the operator. The presenting may e.g. be performed by a device also used for the electrical impedance measurement on the tissue.

The assessed quality may be presented in such a way that conclusions may be drawn, e.g. by a computer or a human operator, on whether to use the impedance measurement or not. It may be convenient if the presenting is, or gives, a direct indication of whether the impedance measurement should be used or not. Thus, if e.g. a human operator perceives the assessed quality, the operator does not need to draw any own conclusions, whereby a source of error is eliminated. The corresponding argument is valid if the assessed quality is presented to e.g. a computer instead of to a human operator. The decision on whether to use the impedance measurement may thus be independent of any entity responsible for making the decision. Thus, e.g. even an untrained operator, human or otherwise, may make the decision. The presenting may e.g. be of a Boolean type where only two different presentations are possible, one indicating "use" and the other indicating "do not use". One specific embodiment might e.g. be using one green and one red light source where a lit green light indicates "use" and a lit red light indicates "do not use", or only one light source might be used where a lit light indicates "use" and if the light is not lit that indicates "do not use", or vice versa. It may, however, be convenient to allow the operator to draw its own conclusions in some cases, since the operator may possess additional information facilitating the making of the decision.

The impedance measurement may be the only measurement intended to be used for diagnosing a condition of the tissue, or it may be one of a plurality of measurements intended to be used in combination for the diagnosing. For example, for making the diagnosing, both a measurement of a tissue region to be diagnosed and a reference measurement of another tissue region on the same or on other tissue may be needed. It may be convenient to use the inventive method for a reference impedance measurement performed on a reference tissue region of the tissue, i.e. a measurement on a tissue region which is not believed to comprise the condition to be diagnosed. More specifically, it may be convenient if the tissue region is a region of apparently normal or healthy tissue in no need of diagnosing. Normal or healthy tissue may be more homogenous and more easily standardised which may facilitate the quality assessment of the measurement. It may be difficult to know how a measurement on abnormal or unhealthy tissue should be, why it may be difficult to assess the quality of the measurement for use in diagnosis. However, at least some parameters of a measurement on a tissue region including tissue which condition is to be diagnosed may be evaluated for assessing the quality of the measurement, why the inventive method may also be relevant to non-reference, i.e. target, measurements.

The subject may be any type of subject, such as an animal or plant subject, dead or alive. It is currently envisioned that the disclosure may be most applicable to live animal, such as human and/or domestic animal, subjects. The tissue may be any type of tissue, such as skin or tissue of internal organs, e.g. cortex. It is currently envisioned that the invention may be most applicable to animal skin. Such skin may be afflicted with many different disorders or lesions that one may want to diagnose, such as different types of skin cancers, e.g. malignant melanoma, squamous cell carcinoma or basal cell carcinoma or precursors thereof such as acitinic keratose or dysplastic nevi, or other malignant or benign conditions, e.g. brought on by ageing, sun damage or collagen composition.

The evaluation algorithm may be any algorithm adapted to assess the quality of the impedance measurement based on the obtained impedance data, such as a trained algorithm. The algorithm may comprise a plurality of parts adapted to perform different functions in the assessing.

The evaluation algorithm may comprise a part adapted to reduce or remove spikes and/or background noise from the obtained impedance data. This part may thus e.g. remove obviously incorrect, outlaying, values. If the data comprises value curves over a continuous or discontinuous frequency spectrum, the curves may e.g. be smoothed by application of this part of the algorithm. The algorithm may take into consideration expected values, e.g. by deleting or adjusting obtained values outside of a predetermined range within which correct impedance values are expected to be. For example, a median filter may be used. Thus, these obviously incorrect or unrepresentative values may not affect any further assessment of the impedance measurement or any tissue condition diagnosing. This part of the evaluation algorithm may be adapted to adjust the impedance data for further evaluation, rather than to reject the whole measurement as being of poor quality.

The evaluation algorithm may comprise a part adapted to reject impedance values, such as magnitude and phase, or whole impedance measurements, which are unrealistic even if they are not spikes, noise or outliers in a statistical sense. For instance, if the subject is a live animal, this part of the evaluation algorithm may e.g. filter away values that are not physiological or are not reasonable in respect of the measured tissue. The evaluation algorithm may thus comprise a part adapted to determine whether the obtained impedance data is physiological/non-physiological. Thus, obviously incorrect or unrepresentative values may not affect any further assessment of the impedance measurement or any tissue condition diagnosing. If the impedance data of a live animal is determined to be non-physiological, the whole measurement may be rejected as being of poor quality instead of only deleting specific values from the data.

The evaluation algorithm may comprise a part adapted to determine whether the impedance data has been obtained from inappropriate tissue. If, e.g., the measurement is supposed to be of normal and/or healthy tissue (e.g. as a reference measurement) this part may reject measurements where the values are not in conformity with a measurement of normal and/or healthy tissue, such as measurements of lesions or abnormalities. If, e.g. the tissue is skin, it may be inappropriate to perform a reference measurement on e.g. ulcerous skin or rashed skin, or on too dry or too moist skin, or on too hard and/or thick skin. It may also be inappropriate to measure some skin types, such as mucous, facial or acral skin, acral skin specifically including the skin of hand palms and foot soles. These types of skin may not be representative of normal healthy skin why they may be a bad choice for a measurement, especially a reference measurement, or they may require an algorithm that is specifically adapted for that type of skin or other tissue. Thus, the evaluation algorithm may comprise a part adapted to determine whether the impedance data has been obtained from acral skin. The phase spectra of measurements of acral skin may have a distinct shape which may motivate a special filter for this type of measurements.

The evaluation algorithm may comprise a part adapted to assess the impedance data based on a plurality of parameters. This part may be called a main classifier. By using a plurality of parameters in combination, a more complex assessment of the data may be performed in order to determine whether the impedance measurement is of good or bad quality. For instance, if impedance values are obtained over an essentially continuous or discontinuous frequency spectrum, the obtained values may be compared with corresponding known values of good quality, e.g. curves over the spectrum may be compared, whereby obtained curves having an essentially different shape and/or other characteristic may be declared of bad quality. Examples of parameters that may be used in this part of the algorithm include, but are not limited to, the variation, e.g. variance or standard deviation, of impedance magnitude; variation, e.g. variance or standard deviation,of impedance phase; absolute value of impedance magnitude; absolute value of impedance phase, skewness (i.e. asymmetry of value distribution) of impedance magnitude, skewness of impedance phase; and variation, e.g. variance or standard deviation, of impedance phase maxima position. Corresponding parameters may also be used, or be used instead. For example, the standard deviation, the variance or any other parameter relating to the variation may be used. One or several parameters may be used for one or several different AC frequencies. Which parameters to use, and at which frequencies, may be decided empirically and/or with the help of a computer program to get the most accurate assessment of the impedance measurement. Thus, the evaluation algorithm may comprise a part for assessing the impedance data based on a plurality of parameters including at least one parameter from the group consisting of variation of magnitude, variation of phase, absolute value of magnitude, absolute value of phase, skewness of magnitude, skewness of phase and variation of phase maxima position.

Any one or several of the above discussed suggested evaluation algorithm parts, or any other conceivable part not specifically discussed here, may be included in the evaluation algorithm of the present disclosure. If more than one part is included, the parts may be applied concurrently or consecutively, or a mixture thereof, and in any order. However, it may be practical to apply them consecutively in the order in which they are discussed above. If the measurement is assessed as being of poor quality by one of the parts, it may be convenient not to apply any following part since the measurement has already been rejected, or any following parts may be applied anyway. It may be convenient to allow the quality assessment to be based on a combined result of all or some of the algorithm parts, or it may be enough if one part says that the quality is poor to reject the measurement.

It is to be understood that a method according to the above aspect of the present disclosure may advantageously be realized in a computer program comprising computer code for performing the method or a computer readable digital storage medium, non-limiting examples of which is a CD, DVD, floppy disk, hard-disk drive, tape cartridge, memory card and an USB memory device, on which computer readable digital medium such a computer program is stored. Such a computer program and storage medium are within the scope of the present invention.

According to another aspect of the present disclosure, there is provided a device for electrical impedance measurement on tissue of a subject, the device comprising: an impedance signal unit arranged to obtain impedance data of a tissue region of said tissue of the subject, said data comprising at least one impedance value measured in said tissue region; an evaluation unit arranged to apply an evaluation algorithm to the obtained impedance data, whereby the quality of the impedance measurement is assessed; and a presenting unit arranged to present the assessed quality of the impedance data such that a decision can be made on whether to use the impedance data for diagnosing a condition of said tissue of the subject.

These three units may all be arranged together in a single entity, e.g. in a communal housing, or form separate entities, or two of the units may be arranged together as a single entity while the third forms a separate entity. Also other, not here discussed, units may be arranged together or separate from the single and/or separate entities.

It may be convenient to arrange at least the impedance signal unit in an arrangement adapted to be handheld to allow e.g. a human operator to easily apply the impedance signal unit against the tissue region, such as the skin of a subject, to obtain impedance data of the tissue region. Also the evaluation unit and/or the presenting unit may be arranged in the same handheld arrangement, in order to simplify the handling of the device.

The inventive device may conveniently be used to perform the inventive method discussed above.

The discussion above relating to the inventive method is also relevant in applicable parts to the inventive device. Reference is made to that discussion.

### Brief Description of the Drawings

Currently preferred embodiments of the present invention will be discussed by means of non-limiting examples with reference to the appended drawings, in which:
Fig 1 is a schematic diagram of a part of an impedance signal unit of an exemplary embodiment of the invention and having five electrodes, rendering ten permutations to four different depths.
Fig 2 is a schematic flow chart of a method of the invention.
Fig 3 is a schematic flow chart of the application of an exemplary evaluation algorithm of the invention.
Fig 4 is a schematic diagram of a device according to an exemplary embodiment of the invention.
Fig 5 is a graph of an impedance measurement of good quality.
Fig 6 is a graph of an impedance measurement of poor quality.
Fig 7 is a graph of another impedance measurement of poor quality.
Fig 8 is a graph of another impedance measurement of poor quality.
Fig 9 is a graph of another impedance measurement of poor quality.
Fig 10 is a graph of an impedance measurement on acral skin.

### Detailed Description of Specific Embodiments of the Invention

The impedance signal unit of the device of the present disclosure may comprise a plurality of electrodes, between which electrodes tissue impedance values may be obtained. If the signal unit comprises more than two electrodes spaced from each other, a plurality of impedance measurement permutations may be obtained of different tissue volumes between the electrodes. If e.g. three electrodes are used, one permutation is obtained between the first and the second electrodes, another permutation is obtained between the second and the third electrodes, and still another permutation is obtained between the first and the third electrodes. A total of three permutations may thus be obtained when using three electrodes. If the three electrodes are linearly equidistantly spaced from each other, the distance between the first and the third electrodes will be approximately double the distance between the first and the second, and the second and the third, electrodes. Impedance values obtained between the first and the third electrodes will thus relate to a bigger tissue volume than values obtained between the first and the second, and the second and the third, electrodes. Impedance values obtained between the first and the second electrodes will relate to a different volume than values obtained between the second and the third electrodes, but the volumes will be roughly of the same size, depending on the homogeneity of the tissue. Impedance values of two different volume sizes may thus be obtained when using three linearly equidistantly spaced electrodes.

If five linearly equidistantly spaced electrodes are used, a total of ten different permutations may be obtained relating to four different volume sizes. In this case, ten impedance magnitude values and ten impedance phase values may be obtained at any frequency, without re-positioning of the five electrodes. If the five electrodes are placed against a surface of a tissue, impedance values may be non-invasively obtained to four different depths of the tissue. Any other number of electrodes may also be considered, such as a number of electrodes between 2 and 10, e.g. 4, 6, 7, 8 or 9 electrodes.

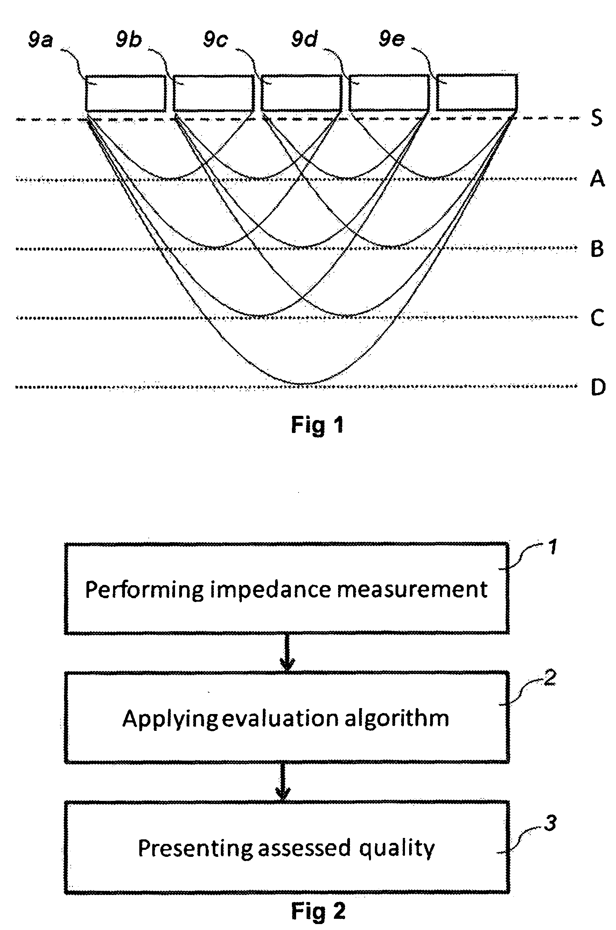

With reference to Fig 1, five linearly equidistantly spaced electrodes 9a-e are placed against the tissue surface of dashed line S, obtaining ten different impedance permutations to four different depths of dotted lines A-D.

The impedance values may be obtained at a plurality of AC frequencies. It may be convenient to obtain the values essentially continuously or discontinuously over an essentially continuous or discontinuous frequency spectrum, such as 0.1-10000 kHz, or 1-3000 kHz, or 1-2500 kHz. Any number of frequencies within these spectra may be used for obtaining the impedance values, such as between 5 and 100, or 10 and 50, or 30 and 40, or about 35, different frequencies. The frequencies may be random within a spectrum, or they may be specifically chosen e.g. empirically based on earlier measurement results, or they may e.g. be equidistantly (linearly or logarithmically) spaced over the spectrum, or chosen in any other way.

It may be important to achieve an adequate electrical contact between the electrodes and the tissue region to be measured. If skin is to be non-invasively measured, it may e.g. be convenient to moisten the skin somewhat with water or another electrically conductive medium before placing the electrodes against the skin surface. Also, it may be convenient to provide the electrodes with small spikes or micro-needles that are able to penetrate the *Stratum corneum* layer of dead skin cells in order to improve the electrical contact with the living tissue. Corresponding measures may also be relevant to take in respect of tissue other than skin.

With reference to Fig 2, an exemplary method in accordance with the present disclosure will now be briefly described. An impedance measurement is performed, step 1, on a tissue region of a subject. The tissue may be skin and the subject may be a human. The measurement may be performed with a handheld impedance measurement device operated by a human operator, such as a staff of a medical or care facility. In the measurement, impedance data of the tissue region is obtained. The data may comprise impedance values of magnitude and phase of a plurality of impedance measurement permutations at a plurality of frequencies. After obtaining the impedance data, an evaluation algorithm is applied, step 2, to the obtained impedance data. By applying the algorithm, the quality of the impedance measurement may be assessed. The algorithm may conclude that the measurement is of good or of poor quality. The assessed quality, e.g. good or poor, is then presented, step 3, enabling a decision to be made whether to use the impedance measurement or not. The assessed quality may e.g. be presented to the human operator or to an automated system. If presented to the human operator, it may e.g. be presented on an LCD display on the handheld device, or in any other fashion. The measurement may be a reference measurement. If the measurement is assessed as of poor quality, it may be convenient to indicate in the presenting 3 why it was of poor quality. This may give guidance to the operator on how to redo the measurement in order for it to be of good quality. It may be convenient to redo the measurement until a measurement of good quality is obtained. A measurement of good quality may then be used e.g. as a reference measurement to be compared with a target measurement on the same tissue, but a different (target) tissue region, in diagnosing a condition of the target tissue region.

The evaluation algorithm may comprise a plurality of different parts which may be applied to the obtained impedance data substantially simultaneously or sequentially or a combination thereof.

A part of the evaluation algorithm may be a pre-processing part. This part may conveniently be applied to the obtained impedance data before any one or several of the other part(s) are applied. The pre-processing part may comprise spike detection and correction, enabling removal of spikes in the impedance magnitude and/or phase angle spectra. Spikes may e.g. be detected with a median filter with an adequate window size. Data points of the filtered data that differ too much from the raw data may be considered to be a spike and may be corrected e.g. by linear interpolation. The pre-processing part may comprise noise reduction, enabling reduction of noise in the impedance magnitude and/or phase angle spectra. The noise reduction may e.g. be made with the use of a Savitsky-Golay smoothing filter. The pre-processing part might not reject any measurements, only adjust them for further assessment.

A part of the evaluation algorithm may be a pre-filter, enabling rejection of measurements that do not fulfil one or a few specific criteria, e.g. cut-offs. The pre-filter may be applied on impedance data that has been corrected/adjusted e.g. by a pre-processing part as discussed above. For example, the magnitude values and/or phase angle values may all be required to fall within a specified magnitude range and a specified phase range, respectively, in order for the measurement not to be rejected. If the measurement is on animal/human skin, the criteria, such as the ranges, may be set such that non-physiological measurements are rejected. Also, a specific criteria may be set for a certain value relating to a specific frequency.

A part of the evaluation algorithm may be an acral filter, enabling rejection of a measurement exhibiting properties typical for measurements made on acral skin, e.g. the skin of palms and foot soles. When performing impedance measurements on human skin, it has been found that acral skin may have properties that makes it unsuitable for the present method. It was found that the phase spectra of acral skin has a distinct shape compared with other skin, why it may be convenient with a special filter to reject measurements on acral skin. The acral filter may e.g. use a Fisher's Linear Descriminant (FLD) classifier with one or a plurality of selected parameters for discrimination between acral and non-acral skin measurements. The acral filter may be applied on impedance data that has been corrected/adjusted e.g. by a pre-processing part as discussed above. If the pre-filter discussed above is also used, the pre-filter may be applied substantially before, after or in parallel with the acral filter.

A part of the evaluation algorithm may be a main classifier. The main classifier may be applied on impedance data that has been corrected/adjusted e.g. by a pre-processing part as discussed above. If the main classifier is used in combination with other parts of the algorithm, such as the pre-filter and acral filter, it may be up to the main classifier to identify and reject those poor quality measurements that are not rejected by those other parts. The main classifier may be the part of the algorithm that rejects most of the poor measurements compared with other parts. If the pre-filter and/or the acral filter discussed above is/are also used, it/they may be applied substantially before, after or in parallel with the main classifier. It may be convenient to allow other parts, such as the pre-filter and/or the acral filter, to be applied to the impedance data before applying the main classifier. That way, the main classifier may not need to be applied to measurements already rejected by other parts of the algorithm, simplifying the quality assessment. It may be convenient to allow measurements that are not rejected by any part of the evaluation algorithm to be assessed as of good quality

The main classifier may e.g. be a Partial Least Squares Discriminate Analysis (PLS-DA) or a Support Vector Machine (SVM) classifier with certain feature parameters. Some of the contemplated parameters are discussed below. The parameters to use for best results may be chosen with the aid of conventional computer programs based on assessed earlier measurements.

The variation of magnitude or phase angle. The variation, e.g. variance or standard deviation or such, of the magnitude or phase of different permutations, or otherwise obtained plurality of impedance values, at one or a plurality of specific frequencies may be fed to the main classifier. The deviation between different permutations at a frequency may give an indication of whether the measurement is of a good quality. If the measurement is a reference measurement, it may be desired to measure on healthy and relatively homogenous tissue, why it may be desirable with a relatively low variation between different permutations. However, it may be expected to have some variation between permutations to different tissue depths.

Absolute value of magnitude or phase angle. If a plurality of permutations are obtained, or otherwise obtained plurality of impedance values at each frequency, the median, or average or such, absolute value of all or some permutations, or such, at one or a plurality of specific frequencies may be fed to the main classifier.

Skewness of magnitude or phase angle. Skewness (third moment of mathematics) is a standard measure of the asymmetry of a distribution, in this case between different permutations or otherwise obtained plurality of impedance values at each frequency. The skewness of different permutations, or such, at one or a plurality of specific frequencies may be fed to the main classifier.

Variation of the position of phase angle maxima between different permutations, or otherwise obtained plurality of impedance phase angle values at each frequency. The maximum of the phase angle for different permutations, or such, may occur at slightly different frequencies. The variation of the positions of the phase maxima may be fed to the main classifier. Generally, it may be desired that the maxima have substantially the same position.

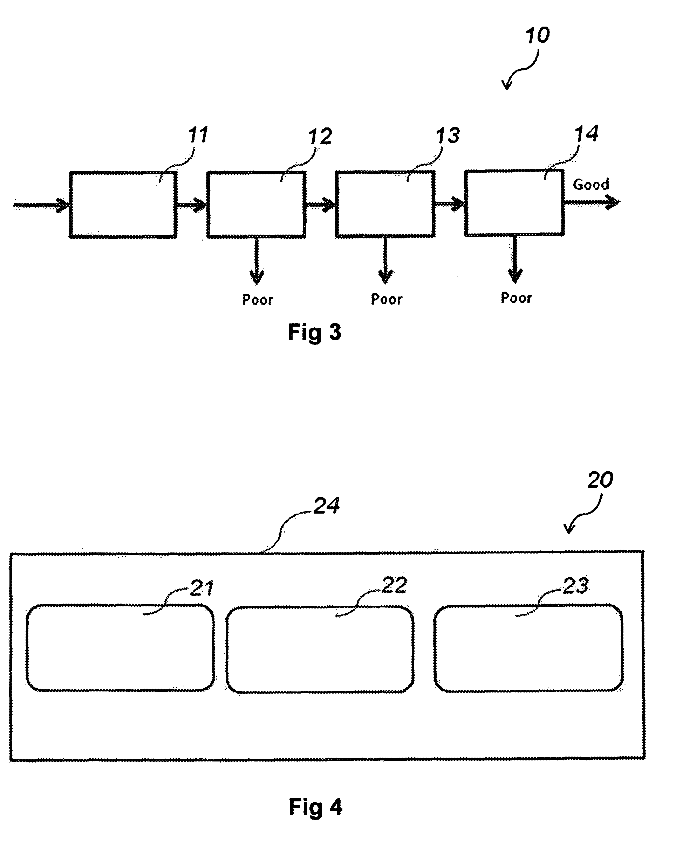

With reference to Fig 3, an exemplary embodiment of the evaluation algorithm will now be briefly discussed. The evaluation algorithm 10 comprises a pre-processing part 11. Obtained impedance data, e.g. from step 1 of Fig 2 on animal skin, may be fed to the pre-processing part 11 where spikes are removed and noise is reduced whereby adjusted impedance data is obtained. The evaluation algorithm 10 also comprises a pre-filter 12. The pre-filter 12 may be applied to the adjusted impedance data from the pre-processing part 11, e.g. rejecting any non-physiological measurements. The evaluation algorithm 10 further comprises an acral filter 13. Provided that the measurement is not rejected by the pre-filter 12, the acral filter 13 is applied to the adjusted impedance data, rejecting any measurements the data of which exhibits properties typical of acral skin. Finally, the evaluation algorithm 10 comprises a main classifier 14. If the impedance data is not rejected by the acral filter, the main classifier 14 is applied to the adjusted impedance data, rejecting or approving the measurement based on a combination of a plurality of parameters. If the impedance data of a measurement is rejected by the pre-filter 12, the acral filter 13 or the main classifier 14, the quality assessment of the measurement is that it is of poor quality. If the impedance data of a measurement is not rejected by any one of the pre-filter 12, the acral filter 13 and the main classifier 14, the quality assessment of the measurement is that it is of good quality.

With reference to Fig 4, an exemplary embodiment of a device in accordance with the present disclosure will now be briefly discussed. A device 20 for electrical impedance measurement on tissue of a subject comprises an impedance signal unit 21. The impedance signal unit 21 is arranged to obtain impedance data of a tissue region of the tissue. The impedance signal unit 21 may e.g. comprise five electrodes arranged to be placed against the tissue as illustrated by Fig 1. In that case, the impedance signal unit 21 obtains impedance data comprising impedance values of impedance magnitudes and phase angles of ten different permutations. Impedance values may relate to any number of different frequencies for each permutation. The device 20 also comprises an evaluation unit 22 arranged to apply an evaluation algorithm to the impedance data obtained by the impedance signal unit 21. The evaluation unit 22 is arranged to be able to communicate with the impedance signal unit 21 such that the obtained impedance data may be transferred to the evaluation unit 22 from the impedance signal unit 21. The evaluation algorithm may e.g. be the evaluation algorithm discussed with reference to Fig 3. The evaluation algorithm may be stored on a medium within the evaluation unit 22. The device 20 also comprises a presenting unit 23 arranged to present the assessed quality, e.g. to an automated or human operator, such that a decision can be made on whether to use the measurement or not. The presenting unit 23 may e.g. comprise an LCD, or other, display for presenting to a human operator. The presenting unit 23 is arranged to be able to communicate with the evaluation unit 22 such that the presenting unit 23 may present the assessment of the evaluation unit 22. Each of the units 21-23 may comprise processing means for performing their respective tasks, or they may share a communal processing means of the device 20. All the units 21-23 may be enclosed within a casing 24, e.g. a casing 24 of a handheld embodiment of device 20.

### Examples

Impedance measurements were performed on regions of skin of a human subject. The measurements are intended to be used as reference measurements for diagnosing a condition of another skin region of the same human subject. A handheld device as illustrated by Fig 4 was used by a human medical staff operator. An impedance signal unit comprising five electrodes giving ten permutations to four different tissue depths (cf. Fig 1) when placed against the skin surface and activated was used.

Impedance data thereby obtained for each measurement contained, as impedance values, the magnitude and phase angles at 35 different frequencies evenly logarithmically spaced over the frequency spectrum 1-2500 kHz. A trained evaluation algorithm, constructed as set out in Fig 3, was applied to the obtained impedance data of each measurement, respectively. The evaluation algorithm had been trained using a large number of measurements manually classified as being of good or poor quality.

In the pre-processing part, spikes are detected with a median filter and removed by linear interpolation and the noise is reduced with a Savitsky-Golay smoothing filter.

In the pre-filter part, any measurements containing impedance values outside the set physiological ranges were classified as of poor quality. The ranges were: absolute value of the magnitude between 0.001 kΩ and 1000 kΩ, and phase angle between 0 and π/2 rad. Additionally, the magnitude at 1 kHz must be at least 10 kΩ, unless the measurement is of the face/head, or the measurement was classified as of poor quality.

In the acral filter, measurements having properties characteristic of measurements made on the skin of palms and foot soles were classified as of poor quality. Three parameters were chosen for feeding an FLD classifier for good discrimination between acral and non-acral reference measurements: the standard deviation of the positions of phase angle maxima of the different permutations, the median of the positions of phase angle maxima of the different permutations, and the mean phase angle at high frequencies.

In the main classifier, each measurement was tested against the combination of a plurality of parameters chosen with the help of software for the best discrimination between reference measurements of good and poor quality, including: the standard deviation of magnitude of the different permutations at 1 kHz, the standard deviation of phase of the different permutations at four specific different frequencies, the absolute value of the magnitude at two specific different frequencies, the median absolute value of the phase angle of the different permutations at four specific different frequencies, the skewness of the magnitude of the different permutations (80^{th} percentile of all frequencies), and the standard deviation of the positions of phase angle maxima of the different permutations. These parameters were fed to an SVM classifier. The output of the classifier was transformed to a p-value and applied to a threshold. Any measurement with a p-value below this threshold was classified as of poor quality.

If a measurement was not classified as of poor quality by anyone of the above discussed parts of the evaluation algorithm, the measurement was classified as of good quality.

An LCD display of the handheld device presented the quality good/poor for each measurement to the operator, whereby the operator could decide whether to use the measurement as a reference measurement for diagnosing a condition of the subject's skin.

Below follows a few typical examples of measurements classified by the evaluation algorithm, with reference to the appended drawings. The magnitude curves are the ones sloping downwards from left to right in the graphs, and the phase angle curves are the ones with a maximum.

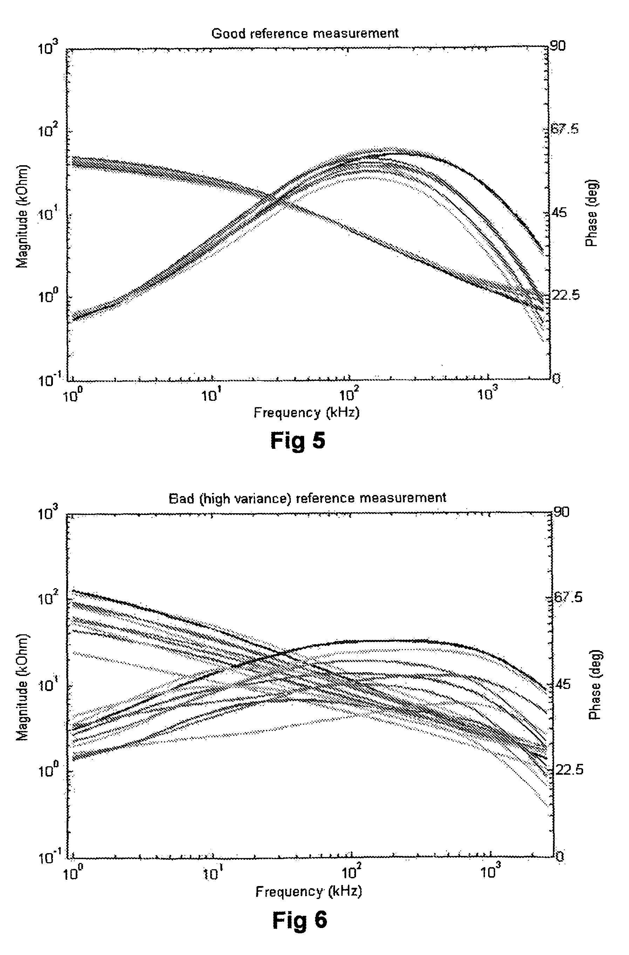

Fig 5 is a graph showing the magnitude and phase of the ten permutations of an impedance measurement of good quality. Notably, there is a low variation between the different permutations, except at high frequencies where the phase curves are split into groups relating to the four tissue depths.

Fig 6 is a graph showing the magnitude and phase of the ten permutations of an impedance measurement of poor quality. Notably, there is a high variation between the different permutations both for phase and magnitude.

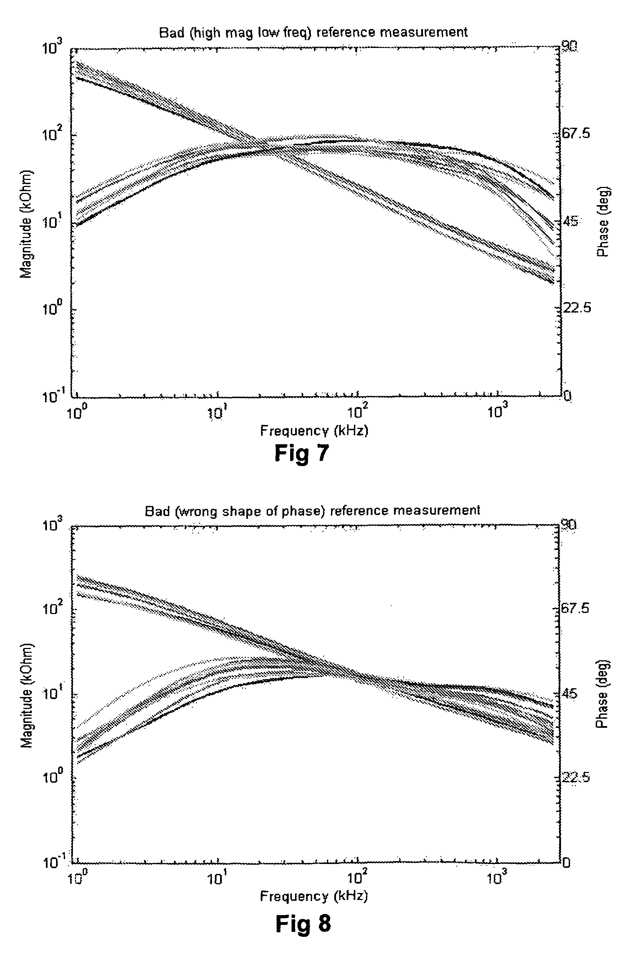

Fig 7 is a graph showing the magnitude and phase of the ten permutations of another impedance measurement of poor quality. Notably, there is a very high magnitude at low frequencies.

Fig 8 is a graph showing the magnitude and phase of the ten permutations of another impedance measurement of poor quality. Notably, the phase curves have a strange shape (cf. the main classifier parameters relating to the absolute values of the phase angle).

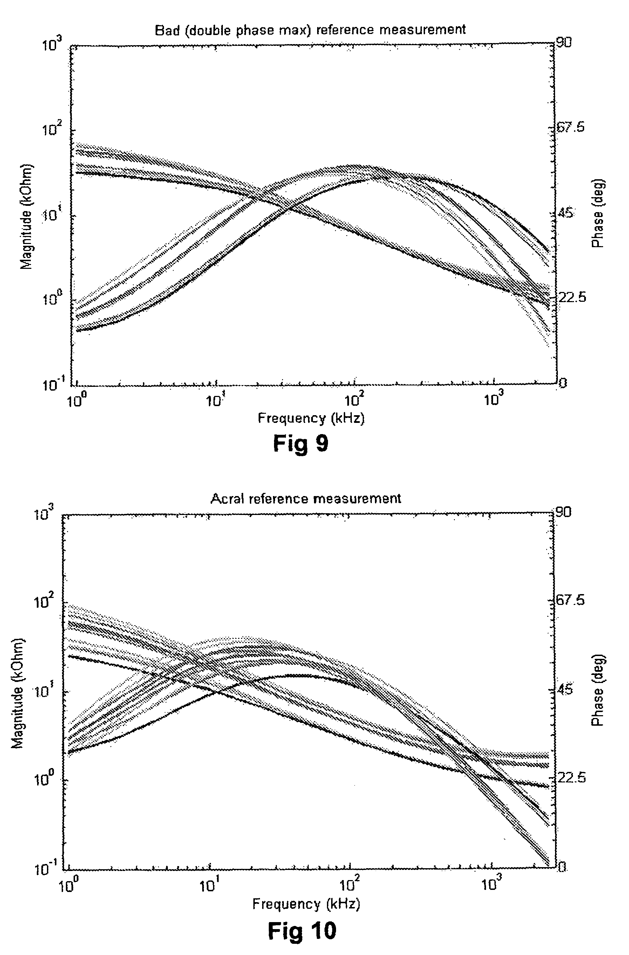

Fig 9 is a graph showing the magnitude and phase of the ten permutations of another impedance measurement of poor quality. Notably, the maximum of the phase angle occurs at different frequencies for different permutations.

Fig 10 is a graph showing the magnitude and phase of the ten permutations of a measurement on acral skin, i.e. of poor quality. Notably, the maximum of the phase angle curves is displaced towards lower frequencies, and the absolute values of the phase angle are very low at high frequencies.

The present invention has above been described with reference to a few embodiments. However, as is readily appreciated by a person skilled in the art, other embodiments than the ones disclosed above are equally possible within the scope of the present invention, as defined by the appended claims.

## Claims

1. A device for quality assesment of an electrical impedance measurement on tissue of a subject wherein the tissue is skin, the device comprising:
an impedance signal unit (21) arranged to obtain impedance data of a tissue region of said tissue of the subject, said data comprising at least one impedance value measured in said tissue region;
an evaluation unit (22) arranged to apply an evaluation algorithm (10) to the obtained impedance data, whereby the quality of obtained impedance data is assessed, the evaluation algorithm comprising:
a pre-processing part (11) configured to reduce spikes and/or background noise from the obtained impedance data whereby adjusted impedance data is obtained;
a pre-filter (12) configured to determine whether the adjusted impedance data is non-physiolgyigal and to reject non-physiological impedance data by comparing the adjusted impedance data with a predetermined impedance data range, wherein data being outside said range are rejected;
an acral filter (13) configured to determine whether the adjusted impedance data has been obtained from acral skin and to reject impedance data which exhibitis properties typical of acral skin; and
a main classifier (14) configured to reject or approve the adjusted impedance data based on a plurality of parameters including at least one parameter from the group consisting of variation of magnitude, variation of phase, absolute value of magnitude, absolute value of phase, skewness of magnitude, skewness of phase and variation of phase maxima position, wherein if the impedance data of a measurement is rejected by the pre-filter (12), the acral filter (13) or the main classifier (14), the quality assessment of the measurement is that it is of poor quality and if the impedance data of a measurement is not rejected by any one of the pre-filter (12), the acral filter (13) and the main classifier (14), the quality assessment of the measurement is that it is of good quality; and
a presenting unit (23) arranged to present the assessed quality of the impedance data such that a decision can be made on whether to use the impedance data for diagnosing a condition of said tissue of the subject.

2. The device of claim 1, wherein at least the impedance signal unit (21) is arranged in an arrangement adapted to be handheld.

3. The device of claim 1 or 2, wherein said impedance data comprisis a plurality of impedance values measured in said tissue region and wherein the plurality of impedance values include magnitude and/or phase at a plurality of frequencies measured at a plurality of tissue depths of the tissue.

4. The device of any one of claims 1-3, wherein said presenting unit (23) is configured to mediate the assessed quality such that it can be perceived by a human operator.

5. The device of any one of claims 1-4, wherein said presenting unit (23) is configured to provide a direct indication of whether the impedance measurement should be used or not.

6. The device of any one of claims 1-5, wherein said impedance measurement is a reference measurement performed on a reference tissue region of said tissue of the subject.

7. A method of assessing the quality of an electrical impedance measurement on tissue of a subject wherein the tissue is skin, the method comprising:
performing the impedance measurement on a tissue region of said tissue of the subject, whereby impedance data is obtained, said data comprising at least one impedance value measured in said tissue region;
applying by an evaluation unit (22) an evaluation algorithm to the obtained impedance data, whereby the quality of the impedance measurement is assessed, the algorithm comprising:
a pre-processing part (11) configured to reduce spikes and/or background noise from the obtained impedance data, whereby adjusted impedance data is obtained;
a pre-filter (12) configured to determine whether the adjusted impedance data is non-physiotogical and to reject non-physiological impedance data by comparing the adjusted impedance data with a predetermined impedance data range, wherein data being outside said range are rejected;
an acral filter (13) configured to determine whether the adjusted impedance data has been obtained from acral skin and to reject impedance data which exhibits properties typical of acral skin; and
a main classifier (14) configured to reject or approve the adjusted impedance data based on a plurality of parameters including at least one parameter from the group consisting of variation of magnitude, variation of phase, absolute value of magnitude, absolute value of phase, skewness of magnitude, skewness of phase and variation of phase maxima position, wherein if the impedance data of a measurement is rejected by the pre-filter (12), the acral filter (13) or the main classifier (14), the quality assessment of the measurement is that it is of poor quality and if the impedance data of a measurement is not rejected by any one of the pre-filter (12), the acral filter (13) and the main classifier (14), the quality assessment of the measurement is that it is of good quality; and
presenting the assessed quality of the impedance measurement such that a decision can be made on whether to use the impedance measurement for diagnosing a condition of said tissue of the subject.

8. The method of claim 7, wherein said impedance data comprisis a plurality of impedance values measured in said tissue region and wherein the plurality of impedance values include magnitude and/or phase at a plurality of frequencies measured at a plurality of tissue depths of the tissue.

## Patentansprüche

1. Vorrichtung zur Qualitätsbeurteilung einer elektrischen Impedanzmessung an Gewebe einer Person, worin das Gewebe Haut ist, wobei die Vorrichtung Folgendes umfasst:
eine Impedanzsignaleinheit (21), die angeordnet ist, Impedanzdaten einer Geweberegion des Gewebes der Person zu erhalten, wobei die Daten zumindest einen Impedanzwert umfassen, der in der Geweberegion gemessen wurde;
eine Bewertungseinheit (22), die angeordnet ist, einen Bewertungsalgorithmus (10) an die erhaltenen Impedanzdaten anzulegen, wobei die Qualität der erhaltenen Impedanzdaten beurteilt wird, wobei der Bewertungsalgorithmus Folgendes umfasst:
einen Vorbehandlungsteil (11), der zur Reduzierung von Spitzen und/oder Hintergrundrauschen von den erhaltenen Impedanzdaten ausgebildet ist, wobei angepasste Impedanzdaten erhalten werden;
einen Vorfilter (12), der ausgebildet ist zu bestimmen, ob die angepassten Impedanzdaten nichtphysiologisch sind, und nicht-physiologische Impedanzdaten durch Vergleich der angepassten Impedanzdaten mit einem vorbestimmten Impedanzdatenbereich zu verwerfen, worin Daten, die außerhalb dieses Bereichs liegen, verworfen werden;
einen Akral-Filter (13), der ausgebildet ist zu bestimmen, ob die angepassten Impedanzdaten von akraler Haut erhalten wurden, und Impedanzdaten, die für akrale Haut typische Eigenschaften zeigen, zu verwerfen; und
einen Hauptklassifikator (14), der ausgebildet ist, die angepassten Impedanzdaten auf Basis einer Vielzahl von Parametern, einschließlich zumindest eines Parameters aus der aus Variation der Größe, Variation der Phase, absolutem Wert der Größe, absolutem Wert der Phase, Schiefe der Größe, Schiefe der Phase und Variation der Phasen-Maxima-Position bestehenden Gruppe, zu verwerfen oder zu genehmigen, worin, wenn die Impedanzdaten einer Messung von dem Vorfilter (12), dem Akral-Filter (13) oder dem Hauptklassifikator (14) verworfen werden, die Qualitätsbeurteilung der Messung eine schlechte Qualität ergibt, und wenn die Impedanzdaten einer Messung weder von dem Vorfilter (12) noch von dem Akral-Filter (13) oder dem Hauptklassifikator (14) verworfen werden, die Qualitätsbeurteilung der Messung eine gute Qualität ergibt; und
eine Präsentationseinheit (23), die angeordnet ist, die beurteilte Qualität der Impedanzdaten so zu präsentieren, dass eine Entscheidung getroffen werden kann, ob die Impedanzdaten zur Diagnose einer Erkrankung des Gewebes der Person verwendet werden sollen.

2. Vorrichtung nach Anspruch 1, worin zumindest die Impedanzsignaleinheit (21) in einer Anordnung angeordnet ist, die in der Hand gehalten werden soll.

3. Vorrichtung nach Anspruch 1 oder 2, worin die Impedanzdaten eine Vielzahl von Impedanzwerten umfassen, die in der Geweberegion gemessen wurden, und worin die Vielzahl von Impedanzwerten Größe und/oder Phase bei einer Vielzahl von Frequenzen, gemessen an einer Vielzahl von Gewebetiefen des Gewebes, umfassen.

4. Vorrichtung nach einem der Ansprüche 1-3, worin die Präsentationseinheit (23) ausgebildet ist, die beurteilte Qualität so zu vermitteln, dass sie von einer menschlichen Bedienungsperson wahrgenommen werden kann.

5. Vorrichtung nach einem der Ansprüche 1-4, worin die Präsentationseinheit (23) ausgebildet ist, einen direkten Hinweis darauf bereitzustellen, ob die Impedanzmessung verwendet werden sollte oder nicht.

6. Vorrichtung nach einem der Ansprüche 1-5, worin die Impedanzmessung eine Referenzmessung ist, die an einer Referenzgeweberegion des Gewebes der Person durchgeführt wurde.

7. Verfahren zur Beurteilung der Qualität einer elektrischen Impedanzmessung an Gewebe einer Person, worin das Gewebe Haut ist, wobei das Verfahren Folgendes umfasst:
Durchführen der Impedanzmessung an einer Geweberegion des Gewebes der Person, wobei Impedanzdaten erhalten werden, wobei die Daten zumindest einen Impedanzwert umfassen, der in der Geweberegion gemessen wurde;
Anlegen, durch eine Bewertungseinheit (22), eines Bewertungsalgorithmus an die erhaltenen Impedanzdaten, wobei die Qualität der Impedanzmessung beurteilt wird, wobei der Algorithmus Folgendes umfasst:
einen Vorbehandlungsteil (11), der zur Reduzierung von Spitzen und/oder Hintergrundrauschen von den erhaltenen Impedanzdaten ausgebildet ist, wobei angepasste Impedanzdaten erhalten werden;
einen Vorfilter (12), der ausgebildet ist zu bestimmen, ob die angepassten Impedanzdaten nichtphysiologisch sind, und nicht-physiologische Impedanzdaten durch Vergleich der angepassten Impedanzdaten mit einem vorbestimmten Impedanzdatenbereich zu verwerfen, worin Daten, die außerhalb dieses Bereichs liegen, verworfen werden;
einen Akral-Filter (13), der ausgebildet ist zu bestimmen, ob die angepassten Impedanzdaten von akraler Haut erhalten wurden, und Impedanzdaten, die für akrale Haut typische Eigenschaften zeigen, zu verwerfen; und
einen Hauptklassifikator (14), der ausgebildet ist, die angepassten Impedanzdaten auf Basis einer Vielzahl von Parametern, einschließlich zumindest eines Parameters aus der aus Variation der Größe, Variation der Phase, absolutem Wert der Größe, absolutem Wert der Phase, Schiefe der Größe, Schiefe der Phase und Variation der Phasen-Maxima-Position bestehenden Gruppe, zu verwerfen oder zu genehmigen, worin, wenn die Impedanzdaten einer Messung von dem Vorfilter (12), dem Akral-Filter (13) oder dem Hauptklassifikator (14) verworfen werden, die Qualitätsbeurteilung der Messung eine schlechte Qualität ergibt, und wenn die Impedanzdaten einer Messung weder von dem Vorfilter (12) noch von dem Akral-Filter (13) oder dem Hauptklassifikator (14) verworfen werden, die Qualitätsbeurteilung der Messung eine gute Qualität ergibt; und
Präsentation der beurteilten Qualität der Impedanzmessung auf eine solche Weise, dass eine Entscheidung getroffen werden kann, ob die Impedanzmessung zur Diagnose einer Erkrankung des Gewebes der Person verwendet werden soll.

8. Verfahren nach Anspruch 7, worin die Impedanzdaten eine Vielzahl von Impedanzwerten umfassen, die in der Geweberegion gemessen wurden, und worin die Vielzahl von Impedanzwerten Größe und/oder Phase einer Vielzahl von Frequenzen, gemessen an einer Vielzahl von Gewebetiefen des Gewebes, umfassen.

## Revendications

1. Dispositif pour l'évaluation de la qualité d'une mesure d'impédance électrique sur un tissu d'un sujet, le tissu étant la peau, le dispositif comprenant :
une unité de signal d'impédance (21) agencée pour obtenir des données d'impédance d'une région de tissu dudit tissu du sujet, lesdites données comprenant au moins une valeur d'impédance mesurée dans ladite région de tissu ;
une unité d'évaluation (22) agencée pour appliquer un algorithme d'évaluation (10) aux données d'impédance obtenues, moyennant quoi la qualité des données d'impédance obtenues est évaluée, l'algorithme d'évaluation comprenant :
une partie de pré-traitement (11) configurée pour réduire les pointes et/ou le bruit de fond dans les données d'impédance obtenues, moyennant quoi des données d'impédance ajustées sont obtenues ;
un pré-filtre (12) configuré pour déterminer si les données d'impédance ajustées sont non physiologiques et rejeter les données d'impédance non physiologiques en comparant les données d'impédance ajustées à une plage de données d'impédance prédéterminée, les données se trouvant en dehors de ladite plage étant rejetées ;
un filtre des extrémités (13) configuré pour déterminer si les données d'impédance ajustées ont été obtenues sur de la peau des extrémités et pour rejeter les données d'impédance qui présentent des propriétés typiques de la peau des extrémités ; et
un classificateur principal (14) configuré pour rejeter ou approuver les données d'impédance ajustées d'après une pluralité de paramètres comprenant au moins un paramètre issu du groupe constitué de la variation de grandeur, la variation de phase, la valeur absolue de la grandeur, la valeur absolue de la phase, l'asymétrie de la grandeur, l'asymétrie de la phase et la variation de la position du maxima de phase, dans laquelle unité d'évaluation, si les données d'impédance d'une mesure sont rejetées par le pré-filtre (12), le filtre des extrémités (13) ou le classificateur principal (14), l'évaluation de qualité de la mesure est que celle-ci est de mauvaise qualité, et si les données d'impédance d'une mesure ne sont rejetées ni par le pré-filtre (12), ni par le filtre des extrémités (13) ni par le classificateur principal (14), l'évaluation de qualité de la mesure est que celle-ci est de bonne qualité ; et
une unité de présentation (23) agencée pour présenter la qualité évaluée des données d'impédance de telle façon qu'une décision puisse être prise quant à la possibilité d'utiliser les données d'impédance pour diagnostiquer une condition dudit tissu du sujet.

2. Dispositif selon la revendication 1, dans lequel au moins l'unité de signal d'impédance (21) est agencée sous forme d'un agencement pouvant être tenu à la main.

3. Dispositif selon la revendication 1 ou 2, dans lequel lesdites données d'impédance comprennent une pluralité de valeurs d'impédance mesurées dans ladite région de tissu et dans lequel la pluralité de valeurs d'impédance comprend la grandeur et/ou la phase à une pluralité de fréquences, mesurées en une pluralité de profondeurs de tissu du tissu.

4. Dispositif selon l'une quelconque des revendications 1 à 3, dans lequel ladite unité de présentation (23) est configurée pour relayer la qualité évaluée de telle façon qu'elle puisse être perçue par un opérateur humain.

5. Dispositif selon l'une quelconque des revendications 1 à 4, dans lequel ladite unité de présentation (23) est configurée pour indiquer directement s'il convient ou non d'utiliser la mesure d'impédance.

6. Dispositif selon l'une quelconque des revendications 1 à 5, dans lequel ladite mesure d'impédance est une mesure de référence effectuée sur une région de tissu de référence dudit tissu du sujet.

7. Procédé d'évaluation de la qualité d'une mesure d'impédance électrique sur un tissu d'un sujet, le tissu étant la peau, le procédé comprenant les étapes consistant à :
effectuer la mesure d'impédance sur une région de tissu dudit tissu du sujet, moyennant quoi des données d'impédance sont obtenues, lesdites données comprenant au moins une valeur d'impédance mesurée dans ladite région de tissu ;
appliquer, au moyen d'une unité d'évaluation (22), un algorithme d'évaluation aux données d'impédance obtenues, moyennant quoi la qualité de la mesure d'impédance est évaluée, l'algorithme comprenant :
une partie de pré-traitement (11) configurée pour réduire les pointes et/ou le bruit de fond dans les données d'impédance obtenues, moyennant quoi des données d'impédance ajustées sont obtenues ;
un pré-filtre (12) configuré pour déterminer si les données d'impédance ajustées sont non physiologiques et rejeter les données d'impédance non physiologiques en comparant les données d'impédance ajustées à une plage de données d'impédance prédéterminée, les données se trouvant en dehors de ladite plage étant rejetées ;
un filtre des extrémités (13) configuré pour déterminer si les données d'impédance ajustées ont été obtenues sur de la peau des extrémités et pour rejeter les données d'impédance qui présentent des propriétés typiques de la peau des extrémités ; et
un classificateur principal (14) configuré pour rejeter ou approuver les données d'impédance ajustées d'après une pluralité de paramètres comprenant au moins un paramètre issu du groupe constitué de la variation de grandeur, la variation de phase, la valeur absolue de la grandeur, la valeur absolue de la phase, l'asymétrie de la grandeur, l'asymétrie de la phase et la variation de la position du maxima de phase, dans laquelle unité d'évaluation, si les données d'impédance d'une mesure sont rejetées par le pré-filtre (12), le filtre des extrémités (13) ou le classificateur principal (14), l'évaluation de qualité de la mesure est que celle-ci est de mauvaise qualité, et si les données d'impédance d'une mesure ne sont rejetées ni par le pré-filtre (12), ni par le filtre des extrémités (13) ni par le classificateur principal (14), l'évaluation de qualité de la mesure est que celle-ci est de bonne qualité ; et
présenter la qualité évaluée pour la mesure d'impédance de telle façon qu'une décision puisse être prise quant à la possibilité d'utiliser la mesure d'impédance pour diagnostiquer une condition dudit tissu du sujet.

8. Procédé selon la revendication 7, dans lequel lesdites données d'impédance comprennent une pluralité de valeurs d'impédance mesurées dans ladite région de tissu et dans lequel la pluralité de valeurs d'impédance comprend la grandeur et/ou la phase à une pluralité de fréquences, mesurées en une pluralité de profondeurs de tissu du tissu.
